# EUROPEAN PATENT APPLICATION

(11) **EP 3 919 094 A1**
(43) Date of publication of application: **08.12.2021**
(21) Application number: 20770230.9
(22) Date of filing: 25.02.2020
(51) Int. Cl.: A61M 1/00, A61B 17/22, A61M 5/315, A61B 17/00

(54) **MEDICAL SYRINGE**

(30) Priority: 13.03.2019 KR 20190028894
(71) Applicant: The Asan Foundation, Seoul 05505 (KR); University Of Ulsan Foundation For Industry Cooperation, Ulsan 44610 (KR); HuBioMed Inc, Seongnam-si, Gyeonggi-do 13516 (KR)
(72) Inventor: CHUNG, Sun, Seongnam-si Gyeonggi-do 13523 (KR); KIM, Kyung Ah, Seoul 05505 (KR); HWANG, Seon Moon, Seoul 05505 (KR); CHOI, Joon Ho, Seoul 05505 (KR); SONG, Yun Sun, Seoul 05505 (KR); LEE, Deok Hee, Seoul 05505 (KR)
(74) Representative: Sander, Rolf
(86) International application number: PCT/KR2020/002680
(87) International publication number: WO 2020/184867

(57) **Abstract**

The present invention may provide a medical syringe comprising: a cylinder including a stopper protruding inward; and a plunger disposed inside the cylinder so as to move back and forth in a first direction, wherein the plunger includes a plurality of protrusions, and the plurality of protrusions are arranged at intervals along the longitudinal direction of the plunger, and based on the first direction, any one of the stopper or the plurality of protrusions is arranged so as to be selectively overlapped according to the rotation of the plunger.

## Description

### [Technical Field]

An embodiment relates to a medical syringe.

### [Background Art]

An angiography field is one of medical fields of treating blood vessel related diseases by inserting a micro syringe needle or the like into a blood vessel. Particularly, when thrombi accumulate in a blood vessel, dangerous and serious diseases such as a stroke, arteriosclerosis, and the like may be caused. Accordingly, the removal of thrombi in a blood vessel is one of significant medical operations.

Here, in addition a method of removing thrombi through thrombolysis, there is a mechanical method of directly inserting a device into a part where thrombi exist and pulverizing or suctioning thrombi. Thrombi may be removed through pulverizing or suctioning by forming a negative pressure or positive pressure inside a blood vessel using a syringe or pump device.

Here, when a pump device is used, a blood vessel may be damaged by an excessive pressure. Also, a pump device connected to a blood vessel has a large volume and is expensive.

In the case of a syringe, when a practitioner releases a plunger of the syringe after suctioning thrombi, the plunger is sucked into a cylinder by a pressure difference such that medical malpractice may occur.

### [Related Art Document]

Korean Patent Registration No. 10-1297661 (published on August 21, 2013)

### [Disclosure]

### [Technical Problem]

The present invention is directed to providing a medical syringe including a simple structure, easily operated, and configured to reduce medical malpractice.

Aspects of the present invention are not limited to the above-stated aspect and other unstated aspects of the present invention will be understood by those skilled in the art from a following disclosure.

### [Technical Solution]

One aspect of the present invention provides a medical syringe including a cylinder including a stopper protruding inward and a plunger disposed inside the cylinder and moved back and forth in a first direction. Here, the plunger includes a plurality of protrusions. The plurality of protrusions are arranged at intervals along a longitudinal direction of the plunger, and the stopper and any one of the plurality of protrusions selectively overlap on the basis of the first direction according to rotation of the plunger.

The plunger may include a body on which the protrusions are arranged and a handle coupled to the body and disposed outside the cylinder.

The body may include a path on which the protrusions are not arranged when viewed in the longitudinal direction of the plunger.

The body may include a first part and a second part which are disposed to perpendicularly intersect each other to form a cross-shaped cross section. Also, the protrusions may be arranged in the second part and spaced apart from the second part to form a path through which the stopper passes.

The body may include a first part and a second part which are disposed to perpendicularly intersect each other to form a cross-shaped cross section, and the protrusions may be arc-shaped plates and have a central angle which is an acute angle.

The protrusions may be disposed only in the second part between the first part and the second part.

The protrusions may include a first protrusion and a second protrusion which are disposed to be spaced apart on the basis of the longitudinal direction of the plunger. Here, the first protrusion may be disposed to be closer to a sealing member of the plunger than the second protrusion. Also, the first protrusion and the second protrusion may each include one side protrusion disposed on one side of the first part and another side protrusion disposed on another side of the first part, and the one side protrusion and the other side protrusion may be disposed to be rotationally symmetrical on the basis of an intersectional center of the first part and the second part.

The one side protrusion and the other side protrusion of the first protrusion may be disposed to be symmetrical on the basis of the first part.

The one side protrusion and the other side protrusion of the first protrusion are disposed to be symmetrical on the basis of the first part.

The one side protrusion may be disposed to protrude from each of a top surface and a bottom surface of the second part, and the one side protrusion disposed on the top surface of the second part and the one side protrusion disposed on the bottom surface of the second part may be disposed to be symmetrical on the basis of the second part.

One side protrusion of the second protrusion may be disposed on a top surface of the second part, and another side protrusion of the second protrusion may be disposed on a bottom surface of the second part.

The second protrusion may include a 2-1 protrusion and a 2-2 protrusion. Here, the 2-1 protrusion may be disposed to be close to the first protrusion rather than the 2-2 protrusion, and a first distance between the first protrusion and the 2-1 protrusion may differ from a second distance between the 2-1 protrusion and the 2-2 protrusion on the basis of the longitudinal direction of the plunger.

The body may include a first part and a second part disposed to perpendicularly intersect each other to form a cross-shaped cross section. Here, the protrusions may be arranged in the second part.

The first part may include a first groove through which the stopper passes in a direction in which the plunger rotates, and the second part may include a second groove through which the stopper passes in the direction in which the plunger rotates.

The first groove may be disposed between a sealing member of the plunger and the protrusion disposed to be closest to the sealing member among the plurality of protrusions.

The second groove may be disposed between the handle of the plunger and the protrusion disposed to be closest to the handle among the plurality of protrusions.

### [Advantageous Effects]

According to an embodiment, since a plunger is fixed after thrombi are extracted, there is provided an advantageous effect of easily performing treatment.

According to an embodiment, there is provided an advantageous effect of easily suctioning thrombi using only a syringe without a large suction device.

According to an embodiment, there is provided an advantageous effect of measuring a pressure inside a human body while suctioning thrombi.

### [Description of Drawings]

FIG. 1 is a view illustrating a medical syringe according to an embodiment;
FIG. 2 is a view illustrating a cylinder;
FIGS. 3 to 6 are views illustrating a plunger;
FIG. 7 is a view illustrating a first protrusion taken along line A-A of FIG. 3;
FIG. 8 is a view illustrating a 2-1 protrusion taken along line B-B of FIG. 3;
FIG. 9 is a view illustrating a 2-2 protrusion taken along line C-C of FIG. 3;
FIG. 10 is a view illustrating a process in which the first protrusion and a stopper overlap each other in a first direction;
FIG. 11 is a view illustrating a handle;
FIGS. 12A and 12B are views illustrating a state of the syringe before suction;
FIGS. 13A and 13B are views illustrating a state of the syringe after suction;
FIGS. 14A and 14B are views illustrating another state of the syringe after suction;
FIGS. 15A and 15B are views illustrating still another state of the syringe after suction; and
FIG. 16 is a view illustrating the medical syringe according to the embodiment which is connected to a manifold.

### [Modes of the Invention]

The aspects, particular advantages, and novel features of the present invention will become apparent from the following detailed description of exemplary embodiments with reference to the attached drawings. Also, the terms used in the specification and the claims should not be limited to general or lexical meanings and should be construed as meanings and concepts coinciding with the technical concept of the present invention on the basis of a principle in which the inventor can appropriately define the concept of the terms to describe the invention in the best manner. Also, in describing the present invention, a detailed description of well-known functions or components of the related art will be omitted when it is deemed to obscure the essence of the present invention.

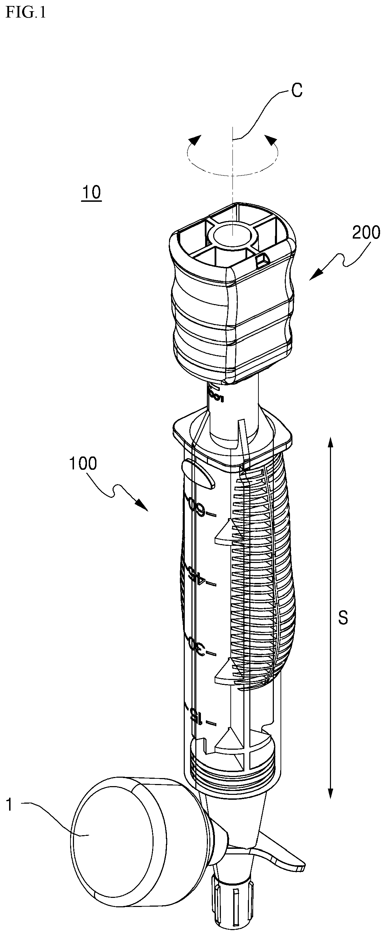

FIG. 1 is a view illustrating a medical syringe 10 according to an embodiment.

Referring to FIG. 1, the medical syringe 10 may include a cylinder 100, a plunger 200, and a negative pressure gauge 1.

The cylinder 100 may include a cylindrical accommodation space therein. The cylinder 100 includes a nozzle at a front, and a rear thereof is opened so that the plunger 200 is inserted thereinto. The plunger 200 is disposed inside the cylinder 100. The plunger 200 moves forward or backward in a first direction S inside the cylinder 100. Also, the plunger 200 is disposed in the cylinder 100 to be rotatable on the basis of an axial center C parallel to the first direction S in the cylinder 100. The negative pressure gauge 1 may be disposed in the cylinder 100. A practitioner may measure a negative pressure inside a blood vessel using the negative pressure gauge 1 during a treatment process.

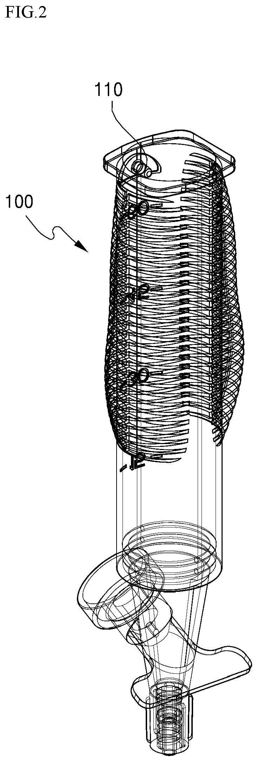

FIG. 2 is a view illustrating the cylinder 100.

Referring to FIG. 2, the cylinder 100 includes a stopper 110. The stopper 110 is disposed to protrude from an inner surface of the cylinder 100. The stopper 110 may be disposed on a periphery of an inlet of the cylinder 100 through which the plunger 200 is inserted.

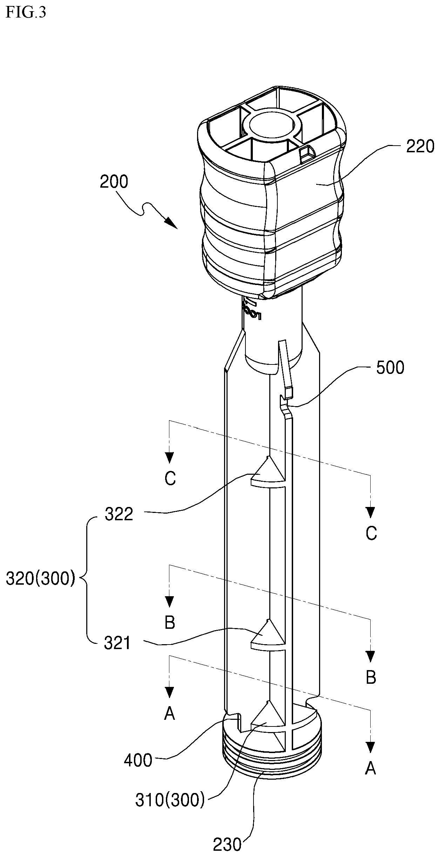

FIGS. 3 to 6 are views illustrating the plunger 200.

Referring to FIGS. 3 to 6, a handle 220 is disposed on one side of a body 210 of the plunger 200. A sealing member 230 may be disposed on the other side of the plunger 200. The practitioner may rotate the plunger 200 while holding the handle 220.

The body 210 may include a first part 211 and a second part 212. The first part 211 and the second part 212 may be planar plates. The first part 211 and the second part 212 may be disposed to vertically intersect with each other so as to form a cross-shaped cross section.

The plunger 200 includes a plurality of protrusions 300. The protrusions 300 are disposed to be selectively overlapped with the stopper 100 according to rotation of the plunger 200. On the basis of the first direction S, the protrusions 300 are restricted by the stopper 110 so as to prevent the plunger 200 from being suctioned into the cylinder 100 due to a pressure difference in treatment.

The protrusions 300 may be disposed only in the second part 212 between the first part 211 and the second part 212. The plurality of protrusions 300 may be arranged at certain intervals along a longitudinal direction of the plunger 200.

The protrusions 300 may include a first protrusion 310 and a second protrusion 320. The first protrusion 310 of the plurality of protrusions 300 is the protrusion 300 disposed to be closest to the sealing member 230. The second protrusion 320 is the protrusion 300 disposed to be relatively close to the handle 220 in comparison to the first protrusion 310. Also, the second protrusion 320 may include a 2-1 protrusion 321 and a 2-2 protrusion 322. The 2-1 protrusion 321 between the 2-1 protrusion 321 and the 2-2 protrusion 322 is the protrusion 300 disposed to be relatively close to the first protrusion 310 and the 2-2 protrusion 322 is the protrusion 300 disposed to be relatively close to the handle 220.

On the basis of the longitudinal direction of the plunger 200, a first distance L1 between the first protrusion 310 and the 2-1 protrusion 321 may differ from a second distance L2 between the 2-1 protrusion 321 and the 2-2 protrusion 322.

The first part 211 may include a first groove 400 through which the stopper 110 passes in a rotation direction of the plunger 200. The first groove 400 may be disposed between the first protrusion 310 and the sealing member 230.

The second part 212 may include a second groove 500 through which the stopper 110 passes in the rotation direction of the plunger 200. The first groove 500 may be disposed between the 2-2 protrusion 322 and the handle 220.

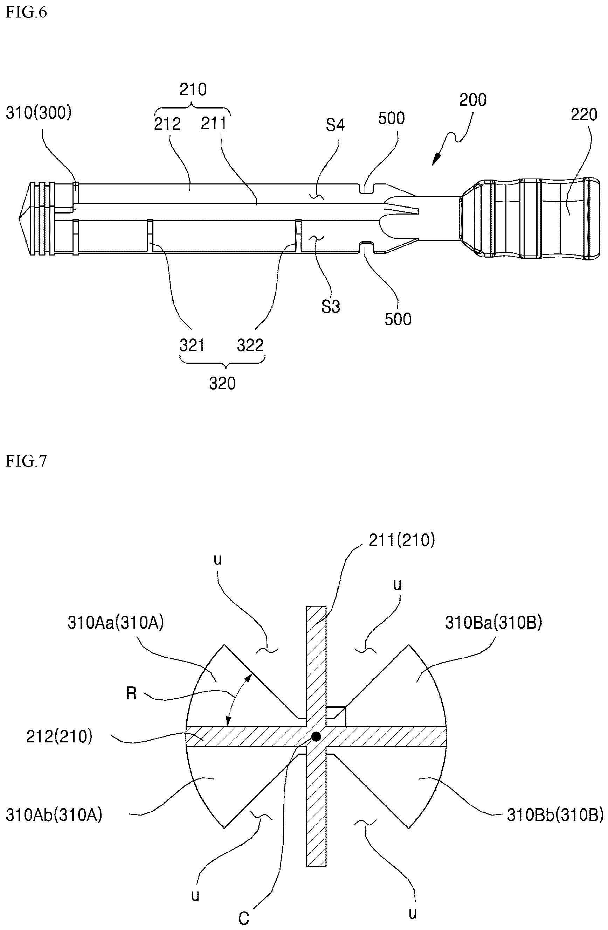

FIG. 7 is a view illustrating the first protrusion 310 taken along line A-A of FIG. 3.

Referring to FIG. 7, the first protrusion 310 may be an arc-shaped plate having a central angle R which is an acute angle. The first protrusion 310 may be disposed on the second part 212 and may be disposed to be spaced apart from the first part 211 to form a path u through which the stopper 110 passes. A plurality of such first protrusions 310 may be present.

For example, the first protrusions 310 may include one side protrusion 310A disposed on one side of the first part 211 and another side protrusion 310B disposed on the other side of the first part 211. On the basis of the axial center C of the first part 211 and the second part 212, the one side protrusion 310A and the other side protrusion 310B may be disposed to be rotationally symmetrical. Also, the one side protrusion 310A and the other side protrusion 310B may be disposed symmetrically on the basis of the first part 211.

The one side protrusion 310A may be disposed to protrude from each of a top surface and a bottom surface of the second part 212. One side protrusion 310Aa disposed on the top surface of the second part 212 and one side protrusion 310Ab disposed on the bottom surface of the second part 212 may be disposed symmetrically on the basis of the second part 212.

The other side protrusion 310B may be disposed to protrude from each of the top surface and the bottom surface of the second part 212. Another side protrusion 310Ba disposed on the top surface of the second part 212 and another side protrusion 310Bb disposed on the bottom surface of the second part 212 may be disposed symmetrically on the basis of the second part 212.

As a whole, the first protrusions 310 may include four protrusions.

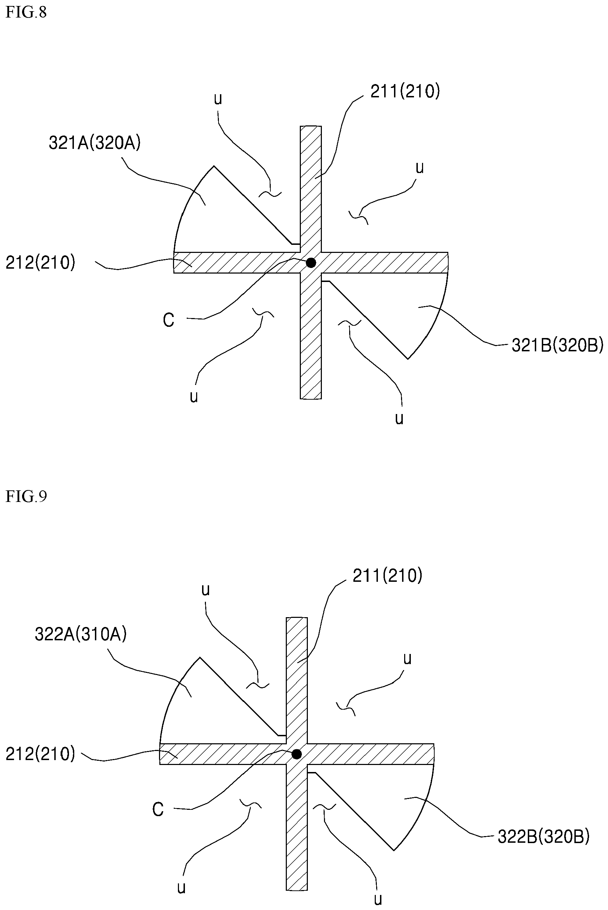

FIG. 8 is a view illustrating the 2-1 protrusion 321 taken along line B-B of FIG. 3.

Referring to FIG. 8, the 2-1 protrusion 321 may be an arc-shaped plate having a central angle R which is an acute angle. The 2-1 protrusion 321 may have the same size and shape as those of the first protrusion 310. The 2-1 protrusion 321 may be disposed on the second part 212 and be spaced apart from the first part 211 to form the path u through which the stopper 110 passes. A plurality of such 2-1 protrusions 321 may be present.

For example, the 2-1 protrusions 321 may include one side protrusion 321A disposed on one side of the first part 211 and another side protrusion 321B disposed on the other side of the first part 211. On the basis of the intersectional center C of the first part 211 and the second part 212, the one side protrusion 321A and the other side protrusion 321B may be disposed to be rotationally symmetrical.

The one side protrusion 321A may be disposed to protrude from the top surface of the second part 212. The other side protrusion 321B may be disposed to protrude from the bottom surface of the second part 212. The 2-1 protrusion 321 has the path u having a large area, through which the stopper 110 passes, in comparison to the first protrusion 310. This is to allow the plunger 200 to smoothly move back and forth. On the other hand, an area of the path u of the first protrusion 310 is relatively smaller than an area of the path u of the second protrusion 320. Here, since the plunger 200 is maximally withdrawn from the cylinder 100 at a position where the first protrusion 310 is held by the stopper 110, this is to allow the first protrusion 310 of the plunger 200 to be easily held by the stopper 110 rather than allowing the plunger 200 to smoothly move back and forth.

FIG. 9 is a view illustrating the 2-2 protrusion 322 taken along line C-C of FIG. 3.

Referring to FIG. 9, the 2-2 protrusion 322 may be an arc-shaped plate having a central angle R which is an acute angle. The 2-2 protrusion 322 may have the same size and shape as those of the first protrusion 310. The 2-2 protrusion 322 may be disposed on the second part 212 and be spaced apart from the second part 212 to form the path u through which the stopper 110 passes. A plurality of such 2-2 protrusions 322 may be present.

For example, the 2-2 protrusions 322 may include one side protrusion 322A disposed on one side of the first part 211 and another side protrusion 322B disposed on the other side of the first part 211. On the basis of the intersectional center C of the first part 211 and the second part 212, the one side protrusion 322A and the other side protrusion 322B may be disposed to be rotationally symmetrical.

The one side protrusion 322A may be disposed to protrude from the top surface of the second part 212. The other side protrusion 322B may be disposed to protrude from the bottom surface of the second part 212. The 2-2 protrusion 322 has the path u having a large area, through which the stopper 110 passes, in comparison to the first protrusion 310. This is to allow the plunger 200 to smoothly move back and forth.

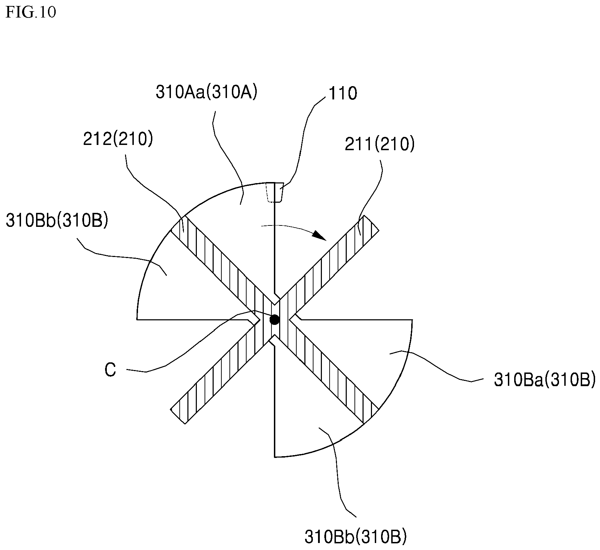

FIG. 10 is a view illustrating a process in which the first protrusion 310 and the stopper 110 overlap each other in the first direction S.

Referring to FIG. 10, when the practitioner manipulates the handle 220 and rotates the plunger 200, the first protrusion 310 and the stopper 110 may be disposed to overlap each other on the basis of the first direction S. Since the first protrusion 310 is held by the stopper 110 in a direction in which the plunger 200 enters, movement of the plunger 200 is restricted. Although not shown in the drawings, when the practitioner manipulates the handle 220 and rotates the plunger 200, the second protrusion 320 and the stopper 110 may also be disposed to be overlapped with each other on the basis of the first direction S.

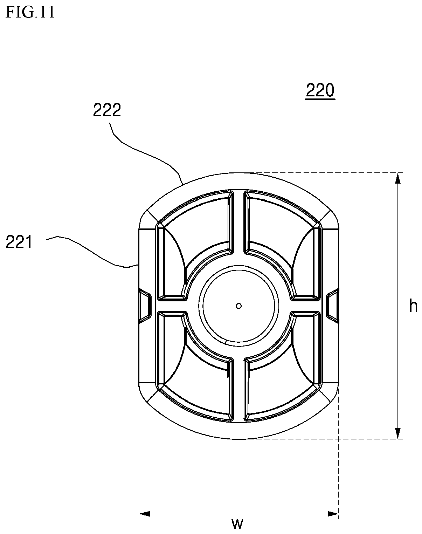

FIG. 11 is a view illustrating the handle.

Referring to FIG. 11, the handle 220 may be formed so that a height h of a lateral surface 221 may be longer than a width w of a longitudinal surface 222. The longitudinal surface 222 may include a curved surface. A shape of the handle 220 as described above has an advantage which allows the practitioner to easily perform rotational manipulation.

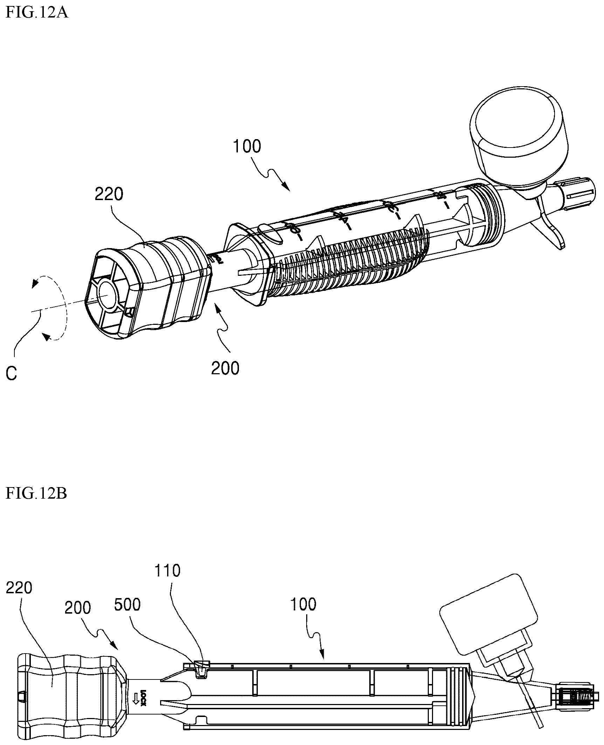

FIGS. 12A and 12B are views illustrating a state of the syringe before suction.

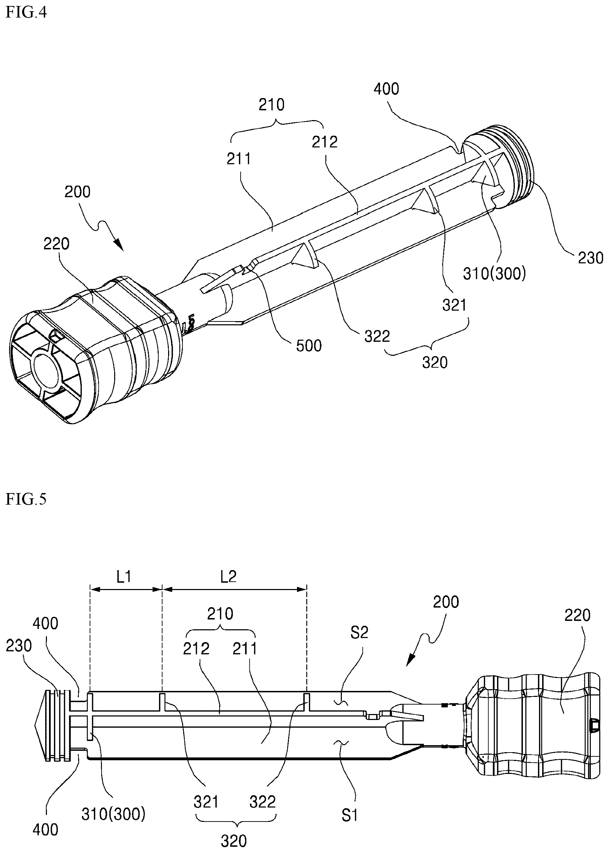

As shown in FIG. 12A, before suction, the plunger 200 is maximally pushed into the cylinder 100. As shown in FIG. 12B, the stopper 110 and the second groove 500 are aligned on the basis of the longitudinal direction of the plunger 200. Referring to FIGS. 5, 12A, and 12B, the stopper 110 is located in the second groove 500 so that the plunger 200 may freely rotate between a first space S1 and a second space S2 within a range of 180° on the basis of the axial center C. Movement of the plunger 200 in the first space S1 is restricted for each section by the first protrusions 310 and the second protrusions 320. On the other hand, movement of the plunger 200 in the second space S2 is not restricted by the first protrusions 310 and the second protrusions 320 and is freely movable in all sections (a suction amount of 0 ml to 60 ml).

Accordingly, according to the needs of a user (doctor), the second space S2 may be selected in order to freely move the plunger 200 such as injecting medicine in one shot and the first space S1 may be selected in order to fix the plunger 200 while adsorbing thrombi.

Also, the second groove 500 may be formed also in the first part 211 so as to configure the plunger 200 to rotate within a range of 360°.

The first protrusions 310 and the second protrusions 320 are located in front of the stopper 110.

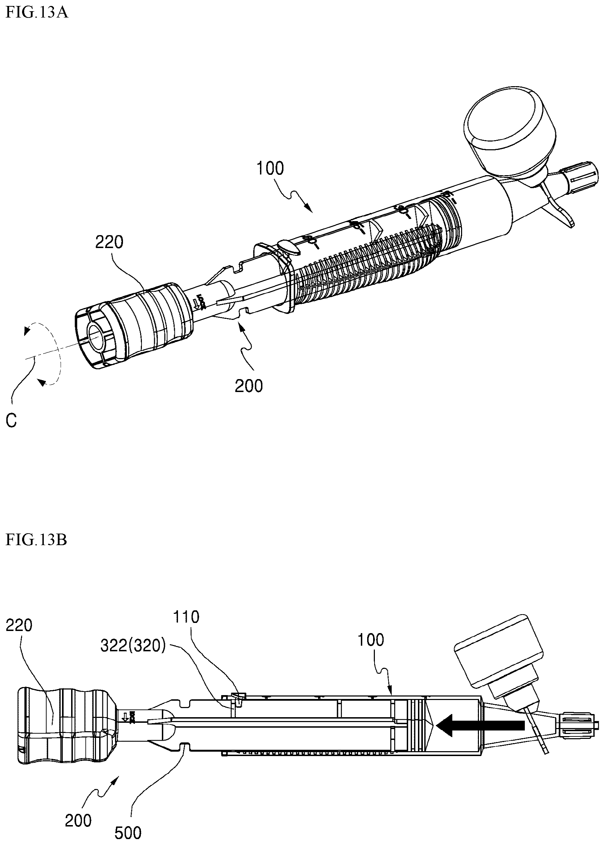

FIGS. 13A and 13B are views illustrating a state of the syringe after suction.

As shown in FIG. 13A, when the practitioner pulls the plunger 200, a certain amount (for example, 30 ml) of thrombi may be suctioned into the cylinder 100. As shown in FIGS. 13A and 13B, when the practitioner pulls the plunger 200 so as to dispose the 2-2 protrusion 322 in the rear of the stopper 110 and then rotates the plunger 200, the 2-2 protrusion 322 is held by the stopper 110 and the plunger 200 is restricted in a direction in which the plunger 200 enters. Accordingly, there is an advantage of preventing the plunger 200 from being suctioned into the cylinder 100 due to a pressure difference even when the user releases the plunger 200 from a hand. Here, the practitioner may check a negative pressure of a blood vessel of a patient using the negative pressure gauge 1.

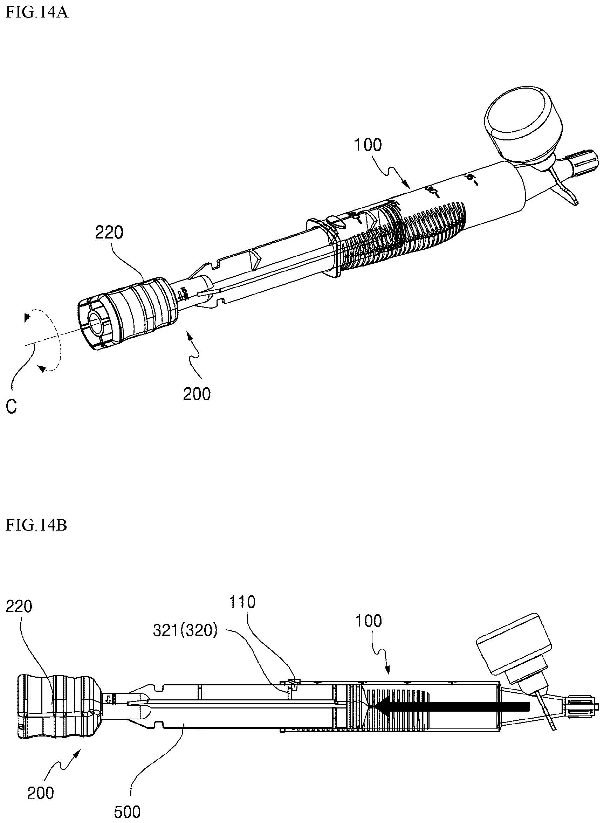

FIGS. 14A and 14B are views illustrating another state of the syringe after suction.

As shown in FIG. 14A, when the practitioner further pulls the plunger 200, a certain amount (for example, 45 ml) of thrombi may be suctioned into the cylinder 100. As shown in FIGS. 14A and 14B, when the practitioner further pulls the plunger 200 so as to dispose the 2-1 protrusion 321 in the rear of the stopper 110 and then rotates the plunger, the 2-1 protrusion 321 is held by the stopper 110 and the plunger 200 is restricted in the direction in which the plunger 200 enters. Accordingly, there is an advantage of preventing the plunger 200 from being suctioned into the cylinder 100 due to a pressure difference even when the user releases the plunger 200 from the hand. Here, the practitioner may check the negative pressure of the blood vessel of the patient using the negative pressure gauge 1.

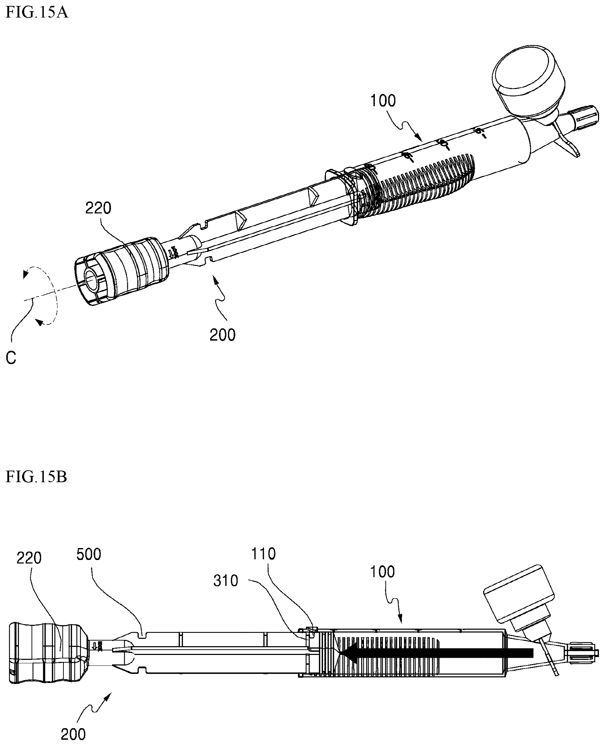

FIGS. 15A and 15B are views illustrating still another state of the syringe after suction.

As shown in FIG. 15A, when the practitioner maximally pulls the plunger 200, a certain amount (for example, 60 ml) of thrombi may be suctioned into the cylinder 100. As shown in FIGS. 15A and 15B, when the practitioner completely pulls the plunger 200 so as to dispose the first protrusion 310 in the rear of the stopper 110 and then rotates the plunger, the first protrusion 310 is held by the stopper 110 and the plunger 200 is restricted in the direction in which the plunger 200 enters. Accordingly, there is an advantage of preventing the plunger 200 from being suctioned into the cylinder 100 due to a pressure difference even when the user releases the plunger 200 from the hand. Here, the practitioner may check a negative pressure of the blood vessel of the patient using the negative pressure gauge 1.

Here, the first protrusion 310, the stopper 110, and the first groove 400 are aligned on the basis of the longitudinal direction of the plunger 200. Since the stopper 110 is disposed to pass through the first groove 400 on the basis of the rotation direction of the plunger 200, as shown in FIGS. 6, 15A, and 15B, the stopper 110 is located in the first groove 400 so that the plunger 200 is freely rotatable between a third space S3 and a fourth space S4 on the basis of the axial center C. Movement of the plunger 200 in the third space S3 is restricted for each section by the first protrusions 310 and the second protrusions 320. On the other hand, movement of the plunger 200 in the fourth space S4 is not restricted by the first protrusions 310 and the second protrusions 320 and is freely movable in all sections (a suction amount of 0 ml to 60 ml).

Accordingly, the stopper 110 may be easily aligned not only with the one side protrusion 310A of the first part 211 but also with the other side protrusion 310B.

Accordingly, according to the needs of the user (doctor), the fourth space S4 may be selected by rotating the plunger 200 in order to freely move the plunger 200 such as injecting medicine in one shot and the third space S3 may be selected in order to fix the plunger 200 while adsorbing thrombi.

Also, the plunger 200 may be configured to be rotatable within a range of 360° by additionally forming the first groove 400 on other parts of the plunger 200.

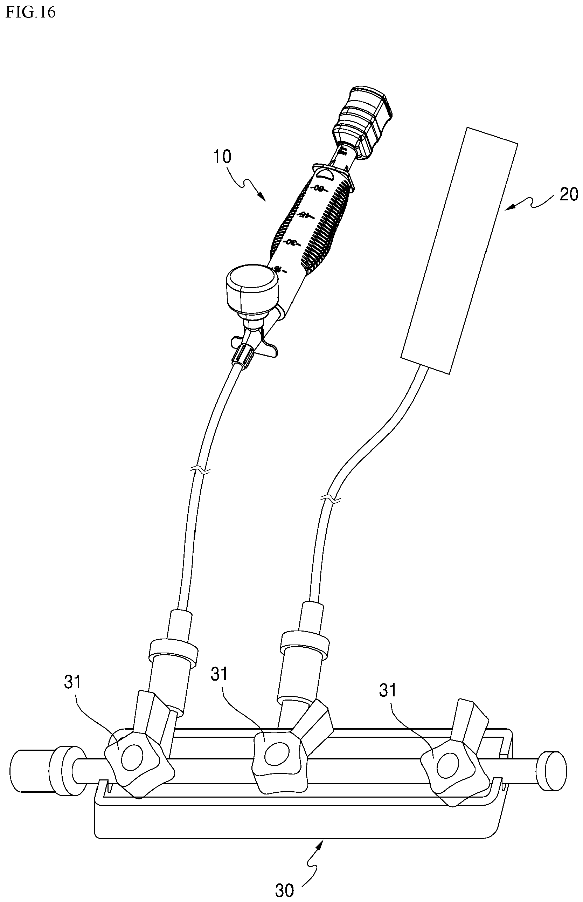

FIG. 16 is a view illustrating the medical syringe 10 according to the embodiment which is connected to a manifold.

Referring to FIG. 16, the medical syringe 10 according to the embodiment may be connected to a manifold 30 including a plurality of valves 31. Devices inserted into a blood vessel may be connected to the manifold 30. A syringe 20 including a positive pressure gauge and injecting medicine into a blood vessel may be connected to the manifold 30. The practitioner may manipulate opening or closing of the valves 31 and perform treatment of injecting medicine or suctioning thrombi by forming a negative pressure or a positive pressure in a blood vessel.

The medical syringe 10 according to one exemplary embodiment of the present invention has been described above in detail with reference to the attached drawings.

It should be noted that the above-described one embodiment of the present invention is merely an example in all aspects and is not intended to be limitative, and the scope of the present invention will be defined by the following claims rather than the above detailed description. Also, it should be construed that all changeable or modifiable forms derived from the meaning and scope of the claims and equivalents thereof are included in the scope of the present invention.

### [Industrial Applicability]

The present invention is applicable to a medical industry.

## Claims

1. A medical syringe comprising:
a cylinder comprising a stopper protruding inward; and
a plunger disposed inside the cylinder and moved back and forth in a first direction,
wherein the plunger comprises a plurality of protrusions,
wherein the plurality of protrusions are arranged at intervals along a longitudinal direction of the plunger, and
wherein the stopper and any one of the plurality of protrusions selectively overlap on the basis of the first direction according to rotation of the plunger.

2. The medical syringe of claim 1, wherein the plunger comprises a body on which the protrusions are arranged and a handle coupled to the body and disposed outside the cylinder.

3. The medical syringe of claim 2, wherein the body comprises a path on which the protrusions are not arranged when viewed in the longitudinal direction of the plunger.

4. The medical syringe of claim 2, wherein the body comprises a first part and a second part which are disposed to perpendicularly intersect each other to form a cross-shaped cross section, and
wherein the protrusions are arranged in the second part and spaced apart from the second part to form a path through which the stopper passes.

5. The medical syringe of claim 2, wherein the body comprises a first part and a second part which are disposed to perpendicularly intersect each other to form a cross-shaped cross section, and
wherein the protrusions are arc-shaped plates and have a central angle which is an acute angle.

6. The medical syringe of claim 5, wherein the protrusions are disposed only in the second part between the first part and the second part.

7. The medical syringe of claim 6, wherein the protrusions comprise a first protrusion and a second protrusion which are disposed to be spaced apart on the basis of the longitudinal direction of the plunger,
wherein the first protrusion is disposed to be closer to a sealing member of the plunger than the second protrusion, and
wherein the first protrusion and the second protrusion each comprise one side protrusion disposed on one side of the first part and another side protrusion disposed on another side of the first part, and the one side protrusion and the other side protrusion are disposed to be rotationally symmetrical on the basis of an intersectional center of the first part and the second part.

8. The medical syringe of claim 7, wherein the one side protrusion and the other side protrusion of the first protrusion are disposed to be symmetrical on the basis of the first part.

9. The medical syringe of claim 8, wherein the one side protrusion and the other side protrusion of the first protrusion are disposed to be symmetrical on the basis of the first part.

10. The medical syringe of claim 9, wherein the one side protrusion is disposed to protrude from each of a top surface and a bottom surface of the second part, and the one side protrusion disposed on the top surface of the second part and the one side protrusion disposed on the bottom surface of the second part are disposed to be symmetrical on the basis of the second part.

11. The medical syringe of claim 8, wherein one side protrusion of the second protrusion is disposed on a top surface of the second part, and another side protrusion of the second protrusion is disposed on a bottom surface of the second part.

12. The medical syringe of claim 11, wherein the second protrusion comprises a 2-1 protrusion and a 2-2 protrusion,
wherein the 2-1 protrusion is disposed to be close to the first protrusion rather than the 2-2 protrusion, and
wherein a first distance between the first protrusion and the 2-1 protrusion differs from a second distance between the 2-1 protrusion and the 2-2 protrusion on the basis of the longitudinal direction of the plunger.

13. The medical syringe of claim 2, wherein the body comprises a first part and a second part disposed to perpendicularly intersect each other to form a cross-shaped cross section,
wherein the protrusions are arranged in the second part,
wherein the first part comprises a first groove through which the stopper passes in a direction in which the plunger rotates, and
wherein the second part comprises a second groove through which the stopper passes in the direction in which the plunger rotates.

14. The medical syringe of claim 13, wherein the first groove is disposed between a sealing member of the plunger and the protrusion disposed to be closest to the sealing member among the plurality of protrusions.

15. The medical syringe of claim 13, wherein the second groove is disposed between the handle of the plunger and the protrusion disposed to be closest to the handle among the plurality of protrusions.
